# EUROPÄISCHE PATENTSCHRIFT

(11) **EP 0 445 068 B1**
(45) Veröffentlichungstag und Bekanntmachung des Hinweises auf die Patenterteilung: **28.07.1993**
(21) Anmeldenummer: 91810072.8
(22) Anmeldetag: 01.02.1991
(51) Int. Cl.: A61F 2/34

(54) **Künstliche Hüftgelenkspfanne**
Artificial hip cup
Prothèse d'acétabule artificielle

(30) Priorität: 02.03.1990 CH 669/90
(43) Veröffentlichungstag der Anmeldung: 04.09.1991
(73) Patentinhaber: GEBRÜDER SULZER AKTIENGESELLSCHAFT, CH-8401 Winterthur (CH)
(72) Erfinder: Zeiler, Günther, Dr. med., W-8501 Schwarzenbruk/Nürnberg (DE); Steiger, Johann Ulrich, Dr. med., CH-8706 Meilen (CH); Koch, Rudolf, CH-8267 Berlingen (CH); Oehy, Jürg, CH-8405 Winterthur (CH)

(56) Entgegenhaltungen:
- EP-A- 0 190 093
- EP-A- 0 308 297
- EP-A- 0 341 199
- FR-A- 2 617 040
- US-A- 4 822 369

## Beschreibung

Die Erfindung betrifft eine künstliche Hüftgelenkspfanne, die aus einer metallischen Aussenschale mit Verankerungshilfen und aus einer Innenschale zur Aufnahme eines Gelenkkugelkopfes besteht, wobei die Innerschale einen in der Montageachse verlanfenden Zentrierstift für die Außenschale aufweist. Eine solche Pfanne ist aus der EP-A-0341 199 bekannt. Wenn metallische Hüftgelenkspfannen als Doppelschalen ausgeführt werden, geschieht dies in der Regel, um eine Verankerung der Aussenschale im Knochengewebe möglich zu machen, ohne die tragende sphärische Fläche der Innenschale für einen Kugelkopf zu beeinträchtigen oder um die Materialeigenschaften Verschleiss- und Notlaufverhalten nicht in einem Teil mit der Gewebefreundlichkeit für das Einwachsen kombinieren zu müssen. Aehnliche Ueberlegungen führen auch zu den Ausführungen mit Kunststoff-Innenschale, wie im US-Patent 4,624,674, wo zusätzlich durch die Teilung der Innenhülse der Kugelkopf in der Innenhülse gefangen ist und mit dieser zusammen in der Aussenhülse durch eine Schnappverbindung verankert wird. Bei all diesen Anwendungen wird die Steifigkeit der Schalen so hoch gewählt, dass gegenüber dem Kugelkopf eine hohe Formbeständigkeit erreicht wird, um die gewünschten Gleiteigenschaften zu erhalten. Die steife Schale hat jedoch den Nachteil, dass sie langfristigen kleinen Veränderungen des stützenden Knochengewebes nicht folgen kann und Belastungsstösse ungedämpft weitergibt.

Hier schafft die Erfindung Abhilfe. Sie löst die Aufgabe, eine künstliche Hüftgelenkspfanne zu schaffen, die mit ihrer Aussenkontur kleinen Veränderungen des stützenden Knochengewebes folgen kann und die für Kraftausgleich zum Knochengewebe sorgt. Gemäss der Erfindung wird die Aufgabe dadurch gelöst, dass die Innenschale metallisch ist, an ihrem Aussenrand ein Radialspiel zur Aussenschale aufweist und sich elastisch mit einem auf ihr befestigten Dämpfungsring radial in einer Nut der Aussenschale abstützt, und dass ferner die Wandstärke der Aussenschale weniger als ein Fünftel der Wandstärke der Innenschale beträgt.

Die abhängigen Ansprüche 2 bis 5 beziehen sich auf vorteilhafte Weiterbildungen der Erfindung. Die Vorteile der Erfindung sind darin zu sehen, dass die Verankerung zwischen Hüftgelenkspfanne und Knochengewebe unabhängig von kleinen Gewebeveränderungen über die ganze eingewachsene Aussenfläche erhalten bleibt, und dass bei schlagartigen Belastungen des Kugelkopfes ein Teil der Belastungsspitze während der Uebertragung auf das Knochengewebe durch die Pfannenschalen abgebaut wird.

Im folgenden wird die Erfindung anhand eines Ausführungs-beispiels beschrieben. Es zeigen:

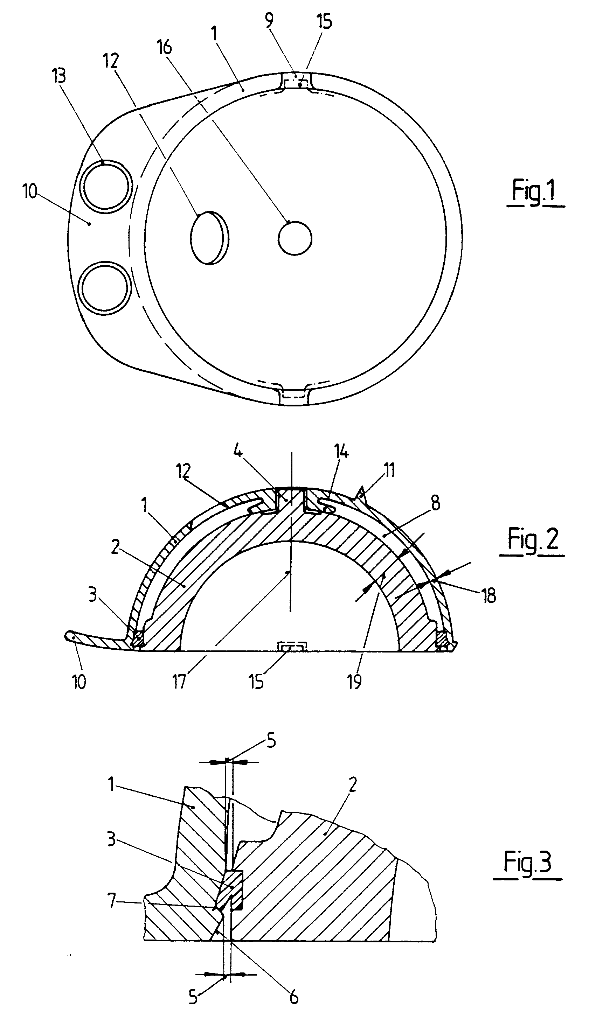

- Fig. 1: Die Ansicht in die Oeffnung einer äusseren Halbschale;
- Fig. 2: einen Längsschnitt durch äussere und montierte innere Halbschale, und
- Fig. 3: einen vergrösserten Ausschnitt aus einem Längsschnitt durch die beiden montierten Halbschalen mit einem verspannten Dämpfungsring zwischen den Halbschalen.

Die Figuren zeigen eine metallische Aussenschale 1 mit einem am Schalenrand angesetzten Randlappen 10, sowie eine metallische Innenschale 2. Die metallische Aussenschale 1 ist über Verankerungshilfen, wie Dorn 11 und durch Knochenschrauben an ihren Befestigungslöchern 12, 13 im Knochengewebe primär verankerbar.

Die Innenschale 2 stützt sich im Bereich des Pols an einer Schulter 14 und mit einem Zentrierstift 4 in einer Zentrierbohrung 16 der Aussenschale 1 ab, während die Schalenränder radial ein Spiel 5 zueinander aufweisen, das ein elastischer, vorgespannter Dämpfungsring 3 überbrückt.

Dabei ist die metallische Aussenschale 1 so dünn gehalten, dass sie sich einerseits nach dem Einwachsen im Knochengewebe kleinen Veränderungen des Knochengewebes elastisch anpassen kann. Andererseits werden Kräfte von der relativ starren metallischen Innenschale 2 nur im Bereich des Pols über einen Zentrierstift 4 und eine Schulter 14 und im Bereich der Schalenränder durch einen Dämpfungsring 3 auf die Aussenschale übertragen, die sich entsprechend den Reaktionskräften im Knochengewebe und entsprechend ihrer Steifigkeit deformiert. Mit der Deformation der metallischen Aussenschale 1 werden Druckspitzen vermieden und wird ein Ausgleich der Spannungen auf einen grösseren Flächenanteil zwischen Knochengewebe und Aussenschale 1 vorgenommen. Der Zentrierzapfen 4 wird bei der Montage der Innenschale 2 in einer Zentrierbohrung 16 längs einer Montageachse 17 geführt, bis der auf der Innenschale 2 befestigte Dämpfungsring 3 in einer Nut 7 der Aussenschale 1 einrastet und die Innenschale 2 sich auf der Schulter 14 der Aussenschale abstützt. Während der Montage wird der elastische Dämpfungsring 3 über eine zur Montageachse 17 geneigte Auflauffläche 6 zusammengepresst bevor er in die Nut 7 zurückspringt. Im montierten Zustand ist der Dämpfungsring 3 soweit vorgespannt, dass er die üblichen Belastungskräfte überträgt, ohne dass ein Radialspiel 5 zwischen Aussenschale 1 und Innenschale 2 total aufgehoben wird. Eine weitere Dämpfungswirkung gegen Belastungsstösse ergibt sich durch eine in Richtung der Montageachse 17 federnde Ausführung der Schulter 14. Um eine wirksame Deformation der Aussenschale 1 zu erzielen, beträgt die Wandstärke 18 der Aussenschale 1 weniger als ein Fünftel der Wandstärke 19 der Innenschale 2. Die Lageänderung der Aussenchale 1 gegenüber der Innenschale 2 wird durch einen Hohlraum 8 zwischen den beiden Schalen ermöglicht und gegebenenfalls begrenzt. Zur Verdrehsicherung sind am Rand der Aussenschale 1 zwei um 180° versetzte Aussparungen 9 angebracht, in die Vorsprünge 15 der Innenschale 2 eintauchen, die in Bezug auf die Montageachse 17 tangential und axial zu den Aussparungen 9 ein Spiel in der Grösse des Radialspiels 5 aufweisen.

In einer bevorzugten Ausführung ist die metallsiche Aussenschale 1 in Titan, die metallische Innenschale 2 in einer Kobaltlegierung und der Dämpfungsring 3 in Polyäthylen ausgeführt.

## Patentansprüche

1. Künstliche Hüftgelenkpfanne bestehend aus einer metallischen Aussenschale (1) mit Verankerungshilfen (11, 12, 13) und aus einer Innenschale (2) zur Aufnahme eines Gelenkkugelkopfes, wobei die Innenschale (2) einen in der Montageachse (17) verlaufenden Zentrierstift (4) für die Aussenschale (1) aufweist, dadurch gekennzeichnet, dass die Innenschale (2) metallisch ist, an ihrem Aussenrand ein Radialspiel (5) zur Aussenschale (1) aufweist und sich elastisch mit einem auf ihr befestigten Dämpfungsring (3) radial in einer Nut (7) der Aussenschale (1) abstützt, und dass die Wandstärke (18) der Aussenschale (1) weniger als ein Fünftel der Wandstärke (19) der Innenschale (1) beträgt.

2. Hüftgelenkpfanne nach Anspruch 1, dadurch gekennzeichnet, dass die Aussenschale (1) auf der Innenseite im Bereich einer Zentrierbohrung (16) eine Schulter (14) aufweist, die die Bewegung der Innenschale (2) beim Einfahren in die Aussenschale (1) abfedert und/oder begrenzt.

3. Hüftgelenkpfanne nach Anspruch 1 oder 2, dadurch gekennzeichnet, dass die Aussenschale (1) und/oder der Dämpfungsring (3) zur Montageachse (17) geneigte Auflaufflächen (6) besitzen, die bei der Montage zur Deformation und anschliessendem Einschnappen des Dämpfungsrings (3) in der Nut (7) führen.

4. Hüftgelenkpfanne nach einem der Ansprüche 1 bis 3, dadurch gekennzeichnet, dass der Dämpfungsring (3) aus Polyäthylen besteht.

5. Hüftgelenkschale nach einem der Ansprüche 1 bis 3, dadurch gekennzeichnet, dass die Aussenschale (1) aus Titan und die Innenschale (2) aus einer Kobaltlegierung bestehen.

## Claims

1. An artificial acetabulum consisting of a metal outer shell (1) having attachment means (11, 12, 13) and of an inner shell (2) for receiving a spherical joint head, the inner shell (2) comprising a centring pin (4) extending in the assembly axis (17) for the outer shell (1),
**characterised in that** the inner shell (2) is metal, on its outer edge comprises a radial clearance (5) towards outer shell (1) and is elastically supported with a damping ring (3) attached thereto radially in a groove (7) of the outer shell (1),
and in that and in that the wall thickness (18) of the outer shell (1) is less than one fifth of the wall thickness (19) of the inner shell (1).

2. An acetabulum according to Claim 1,
characterised in that on the inside in the region of a centring bore (16) the outer shell (1) comprises a shoulder (14), which cushions and/or restricts the movement of the inner shell (2) during insertion into the outer shell (1).

3. An acetabulum according to Claim 1 or 2,
characterised in that the outer shell (1) and/or the damping ring (3) possess abutting surfaces (6) inclined towards the assembly axis (17), which during assembly result in the deformation and subsequent snapping of the damping ring (3) into the groove (7).

4. An acetabulum according to one of Claims 1 to 3,
characterised in that the damping ring (3) is made from polyethylene.

5. An acetabulum according to one of Claims 1 to 3,
characterised in that the outer shell (1) is made of titanium and the inner shell (2) is made of a cobalt alloy.

## Revendications

1. Cavité cotyloïde artificielle se composant d'une coque métallique extérieure (1) comprenant des éléments auxiliaires d'ancrage (11, 12, 13), ainsi que d'une coque intérieure (2) de logement d'une tête sphérique d'articulation, la coque intérieure (2) comportant un téton (4) de centrage de la coque extérieure (1) qui est situé sur l'axe de montage (17), caractérisée en ce que la coque intérieure (2) est métallique, son bord extérieur dispose d'un jeu radial (5) par rapport à la coque extérieure (1) et elle prend appui élastiquement et radialement sur un anneau amortisseur (3) fixé sur elle et disposé dans une rainure (7) de la coque extérieure (1), et en ce que l'épaisseur (18) de la paroi de la coque extérieure (1) est inférieure à un cinquième de l'épaisseur (19) de la paroi de la coque intérieure (2).

2. Cavité cotyloïde selon la revendication 1, caractérisée en ce que la coque extérieure (1) comporte du côté intérieur, dans la région d'un trou de centrage (16), un épaulement (14) qui forme une suspension élastique pour, et/ou qui limite, le mouvement de la coque intérieure (2) lorsque celle-ci se rapproche de la coque extérieure (1).

3. Cavité cotyloïde selon la revendication 1 ou 2, caractérisée en ce que la coque extérieure (1) et/ou l'anneau amortisseur (3) comporte une surface (6) qui forme un plan incliné sur l'axe de montage (17) et qui, lors du montage, provoque la déformation et ensuite l'enclenchement élastique de l'anneau amortisseur (3) dans la rainure (7).

4. Cavité cotyloïde selon l'une des revendications 1 à 3, caractérisée en ce que l'anneau amortisseur (3) est en polyéthylène.

5. Cavité cotyloïde selon l'une des revendications 1 à 3, caractérisée en ce que la coque extérieure (1) est en titane et la coque intérieure (2), en un alliage de cobalt.
